# FASCICULE DE BREVET EUROPEEN

(11) **EP 2 862 556 B1**
(45) Date de publication et mention de la délivrance du brevet: **21.03.2018**
(21) Numéro de dépôt: 14189078.0
(22) Date de dépôt: 15.10.2014
(51) Int. Cl.: A61H 23/00

(54) **Appareil de massage avec tête de massage equipee de doigt de tapotement**
Massagegerät mit einem Massagekopf, der mit einem Klopffinger ausgestattet ist
Massage apparatus having a massage head provided with a tapping finger

(30) Priorité: 17.10.2013 FR 1360117
(43) Date de publication de la demande: 22.04.2015
(73) Titulaire: SEB S.A., 69130 Ecully (FR)
(72) Inventeur: Giraud, Camille, 69001 Lyon (FR); Laranjeira, Laurence, 38440 Moidieu Detourbe (FR); Mandica, Franck, 69340 Francheville (FR)
(74) Mandataire: Guéry-Jacques, Géraldine

(56) Documents cités:
- US-A1- 2005 137 504
- US-A1- 2009 306 561
- US-A1- 2010 331 745
- US-A1- 2012 253 246
- US-A1- 2013 261 516

## Description

La présente invention concerne le domaine des appareils de traitement de la peau, notamment celle du visage. L'appareil selon l'invention permet, pour le moins, le massage de la peau afin de lui donner de la tonicité. L'appareil de massage selon l'invention trouvera son application chez des personnes désireuses de soigner leur esthétique en remodelant, raffermissant et rajeunissant leur peau, notamment celle du visage.

Les appareils de massage de la peau se composent généralement d'un corps muni de moyens de motorisation et d'une tête de massage qui comprend des éléments de massage configurés pour être activés sous l'action des moyens de motorisation, par le biais d'un mécanisme de transmission. Parmi l'art antérieur existant dans ce domaine, il est connu les brevets ou demandes de brevet EP 0 666 071 A1, FR 2 589 726 A1, EP 1 973 510 B1, US 2009/0306561 A1.

Dans le document EP 0 666 071 A1, l'appareil de massage comprend deux éléments de la forme d'une pale qui tournent en sens inversés pour rapprocher lesdites pales qui plissent la peau. Dans une variante, cet appareil de massage comprend un élément filaire souple qui forme une boucle et dont les deux extrémités sont entraînées en rotation en sens inversés, permettant à la partie en boucle de plisser la peau.

Dans le document US 3 626 934 est décrit un appareil de massage de la peau avec deux doigts qui reproduisent un tapotement alternatif selon une direction axiale de chaque doigt vers la peau. Le système présente des doigts sous forme de marteaux avec une grande surface de contact et dont seulement un marteau sur deux vient en contact avec la peau.

De tels appareils de massage permettent donc de réaliser un massage de type « rouler » ou type « tapotement ». Cependant ce type de massage n'est pas le seul permettant d'assurer un rajeunissement et/ou un raffermissement de la peau du visage, notamment, de sorte qu'il est apparu le besoin d'un appareil susceptible de pouvoir réaliser ce type de massage par tapotement ou stimulation régulier et adapté aux zones petites, sensibles et courbées du visage. Ainsi, l'objet de l'invention est de proposer un appareil de massage permettant de procéder à un massage par tapotements de manière à reproduire au mieux le geste du professionnel de l'esthétique et de façon la plus régulière possible un massage qui serait réalisé par tapotement au moyen de deux doigts, tout en permettant d'atteindre une fréquence de tapotement et une durée de massage difficiles voire impossibles à atteindre manuellement et en évitant des sensations désagréables lors de l'application de l'appareil de massage sur la peau, notamment sur la peau du visage.

Afin d'atteindre cet objectif, l'invention concerne un appareil de massage pour le visage comprenant :
- une tête de massage qui comprend :
   - un élément d'appui destiné à venir contre le visage et qui définit une surface d'appui,
   - au dessus de l'élément d'appui au moins un doigt de massage, chacun comprenant une tête de travail destinée à venir au contact du visage et chacun étant mobile entre :
      - une position de rétraction dans laquelle la tête de travail est située en deçà de la surface d'appui vers l'intérieur de la tête de massage,
      - une position d'extension dans laquelle la tête de travail est située au delà de la surface d'appui vers l'extérieur de la tête de massage,
   - des moyens de manoeuvre de chaque doigt de massage adaptés pour déplacer chacun des doigts de massage entre ses positions d'extension et de rétraction de manière alternative,
- et un boitier d'entraînement qui porte la tête de massage et qui comprend un moteur électrique actionnant des moyens d'entrainement adaptés pour transmettre le mouvement du moteur électrique aux moyens de manoeuvre,
- caractérisé en ce que le boîtier d'entrainement est situé à l'opposé des têtes de travail par rapport à l'élément d'appui.

Un tel appareil de massage permet, par le tapotement assuré par le doigt de massage, de stimuler la circulation sanguine autour des yeux pour réduire les cernes / poches. L'appareil de massage peut également être utilisé pour traiter les ridules et les rides du visage, notamment au niveau du sillon naso-génien, en stimulant la circulation sanguine, ralentie par le plissement de la peau au niveau des rides, pour relancer le métabolisme, en particulier la production des éléments constitutifs de la peau.

Selon une forme préférée de réalisation de l'invention, la tête de massage comprend au moins deux doigts de massage.

La mise en oeuvre de deux doigts de massage mobiles de manière alternative permet de reproduire le geste de massage par tapotement réalisé au moyen de deux doigts d'une main. La surface d'appui permet de parfaitement positionner la tête de massage par rapport à la zone à masser notamment en imposant la distance entre les têtes de travail et la zone tapotée de manière à éviter tout risque d'inconfort. La régularité des forces d'appuis par les doigts est par cette surface d'appui encore améliorée sur toute la durée de traitement. En effet, un traitement peut durer par exemple 10 à 20 minutes et l'utilisateur, en appuyant l'appareil via cette surface d'appui pendant tout le traitement, ressentira moins de fatigue pendant la tenue manuelle de l'appareil, et sa tenue sur la durée sera aussi plus précise. Par ailleurs, l'entrainement par moteur électrique permet d'atteindre une fréquence et une durée de massage qu'il ne serait pas possible d'atteindre à la main. De plus, l'entrainement motorisé associé à la surface d'appui permet de garantir une grande reproductibilité du massage réalisé.

Selon une variante de la forme préférée de réalisation, les moyens de manoeuvre sont adaptés pour coordonner le déplacement des doigts de massage de manière que lorsque l'un des doigts de massage est en position d'extension l'autre est en position de rétraction et réciproquement.

Selon une autre caractéristique de l'invention, le boitier d'entraînement est situé à l'opposé des têtes de travail par rapport à l'élément d'appui et sous ce dernier. Une telle configuration rend l'appareil de massage selon l'invention particulièrement adapté pour réaliser un massage par tapotement des régions des pommettes situées sous les yeux.

Selon encore une autre caractéristique de l'invention, l'élément d'appui comprend une surface d'appui concave. Une telle forme de l'élément d'appui permet de bien épouser la forme des pommettes lors d'un massage des régions situées sous les yeux. La régularité des forces d'appuis par les doigts est par ce biais encore améliorée sur toute la durée de traitement.

Selon une autre caractéristique de l'invention, l'élément d'appui comprend une surface d'appui plane.

Selon une caractéristique de l'invention, l'élément d'appui comprend une surface d'appui lisse. Une telle surface lisse permet de déplacer la tête de massage par rapport au visage tout en maintenant le contact avec la peau de sorte que la tête de massage glisse sur la peau, les doigts de massage assurant le massage par tapotement.

Selon une autre caractéristique de l'invention, les doigts de massage s'étendent en partie au moins à l'extérieur d'un corps creux entourant en partie au moins les moyens de manoeuvre.

Selon une autre caractéristique de l'invention, l'appareil de massage est adapté pour assurer à chacun des doigts de massage une fréquence de déplacement supérieure ou égale à 2,5 Hz. De telles fréquences de massage difficiles voire impossible à réaliser manuellement permettent d'obtenir une stimulation optimale alliée à une impression de bien être ainsi qu'à un confort d'utilisation, sans douleur.

Selon encore une autre caractéristique de l'invention, chaque tête de travail possède, entre sa position de rétraction et sa position d'extension, une amplitude de déplacement comprise entre 5 mm et 15 mm. Une telle amplitude de déplacement permet d'assurer un massage tout à la fois efficace et agréable.

Selon une caractéristique de l'invention, en position d'extension chaque tête de travail est saillante par rapport à la surface d'appui d'une distance comprise entre 2 mm et 10 mm. Une telle extension permet un massage efficace sans toutefois appuyer trop fortement sur la peau lors de l'impact avec celle-ci.

Selon une autre caractéristique de l'invention, chaque tête de travail comprend une surface de travail convexe. Une telle forme de la surface de travail permet d'obtenir un confort de massage dans la mesure où la peau n'est pas sollicitée de la même manière sur toute la surface de la tête de travail de sorte que l'impact de la tête de travail sur la peau n'est pas douloureux. De plus, la forme convexe de chaque surface de travail permet d'atteindre des zones étroites et de visualiser l'impact de chaque tête de travail sur la peau.

Selon une variante de l'invention, chaque tête de travail comprend une surface de travail plane. Cette variante permet de stimuler une zone plus large que dans le cas d'une surface de travail convexe et exerçant une pression répartie sur la peau.

Selon une caractéristique de l'invention, chaque tête de travail comprend une surface de travail rigide. Une telle surface rigide permet de transmettre l'intégralité de l'effort de massage à la peau. La surface de travail ou encore la tête de travail en elle-même peut alors être réalisée par exemple en matériau polymère rigide. La surface de travail peut aussi être réalisée en métal. La tête de travail peut également être réalisée sous la forme d'une bille en métal. Une telle bille en métal présente l'avantage d'offrir une sensation de fraicheur au contact. Selon une variante de réalisation l'appareil conforme à l'invention peut alors comprendre pour chaque tête de travail un capuchon souple amovible pouvant se fixer sur la bille de métal et permettant de modifier la rigidité de la surface de travail.

Selon une autre caractéristique de l'invention, chaque tête de travail comprend une surface de travail élastiquement déformable. La surface de travail peut alors être réalisée en élastomère.

Selon une caractéristique de l'invention, l'appareil de massage comprend des moyens d'application d'un courant électrique comprenant au moins une électrode qui est destinée à être en contact avec la peau et qui est raccordée à une unité de génération d'un courant et/ou d'une tension électriques. La mise en oeuvre d'une telle électrode permet l'application de faibles courants sur la peau qui induisent des phénomènes d'électrophorèse ou de iontophorèse favorisant la pénétration de principes actifs appliqués sur la peau préalablement et/ou pendant le massage.

Selon une variante de cette caractéristique, l'élément d'appui porte ou constitue au moins une électrode.

Selon une variante de cette caractéristique, au moins une tête de travail porte au moins une électrode.

Selon une forme de réalisation de l'invention, l'appareil de massage comprend des moyens de diffusion de lumière en direction du visage. La mise en oeuvre de tels moyens de diffusion de lumière permet d'effectuer un traitement de luminothérapie et/ou d'activer des principes actifs appliqués sur la peau préalablement et/ou pendant le massage.

Selon une caractéristique de cette forme de réalisation, les moyens de diffusion de lumière comprennent au moins une source de lumière et au moins un système optique de diffusion comprenant une face de sortie destinée à être orientée vers le visage.

Selon une variante de cette caractéristique, l'élément d'appui comprend une face de sortie de la lumière.

Selon une autre variante de cette caractéristique, l'une au moins des têtes de travail comprend une face de sortie de la lumière.

Selon une autre forme de réalisation de l'invention, l'appareil de massage comprend des moyens d'application ou de distribution de produit cométique. La mise en oeuvre de tels moyens d'application permet de déposer sur la peau un produit cosmétique préalablement et/ou pendant le massage.

Selon une caractéristique de cette forme de réalisation, les moyens d'application ou distribution de produit cosmétique comprennent au moins un capuchon qui comprend un tampon imbibé de produit cosmétique et qui est adapté de manière amovible sur un doigt de massage.

Selon une autre caractéristique de cette forme de réalisation, les moyens d'application ou distribution de produit cosmétique comprennent un réservoir de produit cosmétique et au moins une buse de distribution raccordée à un système de prélèvement de produit dans le réservoir, qui peut être par exemple une pompe.

Selon une variante de cette dernière caractéristique, les moyens d'application ou distribution de produit cosmétique comprennent au moins une buse de distribution située dans une tête de travail ou dans l'élément d'appui.

Selon une caractéristique de l'invention, la tête de massage est adaptée de manière amovible sur le boitier d'entrainement. Le caractère amovible de la tête de massage permet d'utiliser plusieurs têtes de massage interchangeables avec un même boitier.

Selon une variante de cette caractéristique, la tête de massage comprend des moyens d'identification et le boiter d'entrainement comprend des moyens de reconnaissance des moyens d'identification raccordés à une unité de commande adaptée pour contrôler le fonctionnement de l'appareil de massage en fonction de la tête de massage reconnue. La mise en oeuvre d'un tel système d'identification permet d'automatiser le réglage du fonctionnement de l'appareil de massage de sorte que l'utilisateur n'a pas à s'en préoccuper.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

Par ailleurs, diverses autres caractéristiques de l'invention ressortent de la description annexée effectuée en référence aux dessins qui illustrent des formes non limitatives de réalisation d'un appareil de massage selon l'invention.
- La figure 1 est une coupe schématique d'un appareil de massage selon l'invention.
- La figure 2 est une perspective de la tête de massage amovible équipant l'appareil de massage illustré à la figure 1.
- La figure 3 est une perspective partiellement arrachée de la tête de massage illustrée à la figure 2.
- La figure 4 est une coupe schématique d'une autre forme de réalisation d'une tête de massage amovible pour un appareil de massage selon l'invention.
- La figure 5 est une perspective partiellement arrachée de la tête de massage illustrée à la figure 4.
- La figure 6 est une perspective partiellement arrachée d'une autre forme de réalisation d'une tête de massage amovible pour un appareil de massage selon l'invention.
- La figure 7 est une élévation en vue de côté schématique d'une autre forme de réalisation d'une tête de massage amovible pour un appareil de massage selon l'invention.

Il est à noter que sur ces figures les éléments structurels et/ou fonctionnels communs aux différentes variantes peuvent présenter les mêmes références.

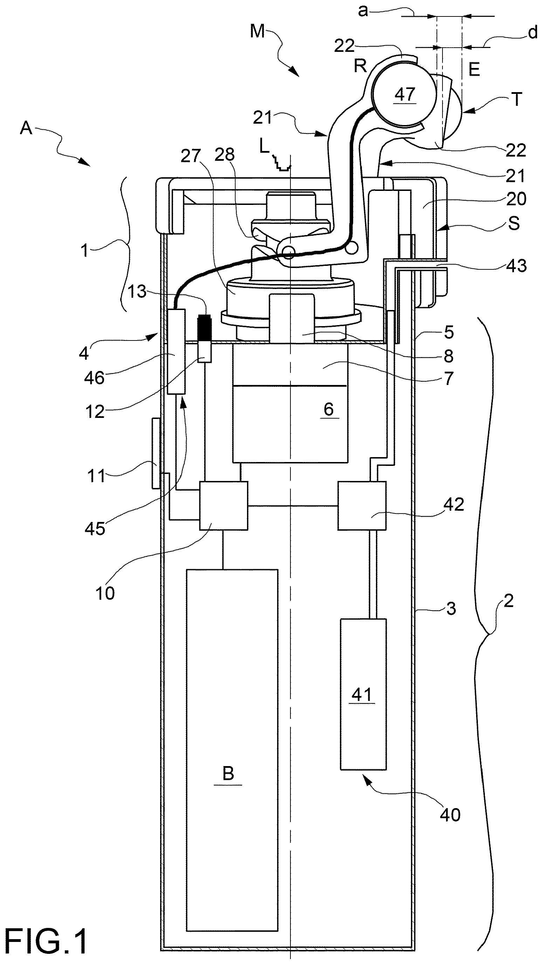

Un appareil de massage selon l'invention, tel qu'illustré à la figure 1 et désigné dans son ensemble par la référence A, comprend une tête de massage 1 adaptée de manière amovible sur un boîtier d'entraînement 2. La tête de massage 1 est conçue pour exercer une action mécanique sur la peau du visage d'un utilisateur par l'intermédiaire d'éléments de massage M entraînés par un moteur électrique.

À cet effet, le boîtier d'entraînement 2 comprend un corps allongé 3 de forme généralement cylindrique qui comprend au niveau d'une de ses extrémités 4 des moyens d'adaptation 5 de manière amovible de la tête de massage 1. Les moyens d'adaptation 5 sont, selon l'exemple illustré, formés par un fourreau à l'intérieur duquel la tête de massage 1 est en partie engagée.

Le boîtier d'entraînement 2 comprend, à l'intérieur du corps 3, un moteur électrique 6 qui actionne des moyens d'entraînement 7 adaptés pour transmettre le mouvement du moteur électrique aux éléments de massage de la tête de massage 1. Selon l'exemple illustré, les moyens d'entraînement 7 comprennent un réducteur non représenté qui entraîne un arbre de sortie 8 accessible au niveau des moyens d'adaptation 5 du boîtier d'entraînement 2.

Le moteur électrique 6 est piloté par une unité de commande 10 alimentée par un bloc de batterie B disposé à l'intérieur du corps 3. Bien entendu, l'alimentation électrique de l'unité de commande 10 pourrait également être effectuée directement à partir du réseau électrique par l'intermédiaire d'un transformateur. L'unité de commande 10 est en outre raccordée à une interface de commande manuelle 11 accessible depuis l'extérieur du corps 3. L'interface de commande manuelle 11 peut par exemple comprendre un interrupteur marche-arrêt et/ou des moyens de sélection manuelle de programmes de fonctionnement.

Le boîtier d'entrainement 2 comprend aussi des moyens de reconnaissance 12 qui sont raccordés à l'unité de commande 10 et qui sont adaptés pour lire des moyens d'identification 13 portés par la tête de massage 1. L'unité de commande 10 est alors adaptée pour contrôler le fonctionnement de l'appareil de massage A en fonction de la tête de massage 1 telle que reconnue suite à la lecture des moyens d'identification 13. Le contrôle du fonctionnement de l'appareil de massage A peut notamment consister en une détermination de la vitesse de rotation du moteur électrique 6 de manière qu'elle soit adaptée au massage devant être réalisé par les éléments de massage M. Les moyens d'identification 13 peuvent, par exemple, être constitués par une puce RFID tandis que les moyens de reconnaissance 12 seront adaptés pour lire une telle puce RFID. Bien entendu, les moyens d'identification 13 et de reconnaissance 12 pourraient être réalisés de toute autre manière appropriée comme par exemple sous la forme d'un système d'identification par contact mécanique ou électrique ou encore sous la forme d'un système d'identification magnétique mettant en oeuvre des aimants permanents et des interrupteurs à lame souple (ILS) également appelés interrupteurs Reed.

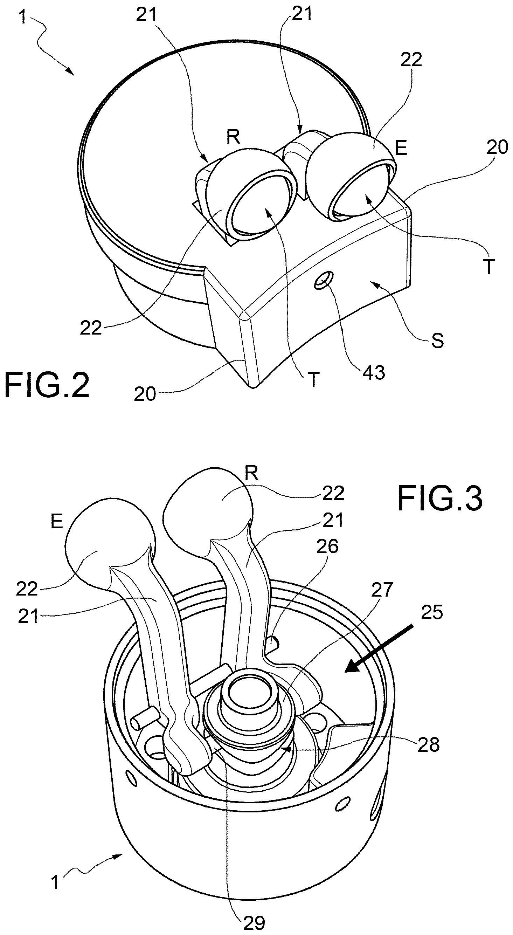

Selon l'invention, la tête de massage 1 est conçue pour réaliser un massage par tapotement. À cet effet, la tête de massage 1 comprend, comme le montre la figure 2 un élément d'appui 20 qui est destiné à venir contre le visage pour définir une distance de travail. Ainsi, l'élément d'appui 20 définit une surface d'appui S qui forme une surface de référence. Comme illustré sur les figures et à l'évidence de l'homme du métier, l'élément d'appui 20 se trouve sur la paroi verticale du boîtier en son extérieur, c'est-à-dire que la surface d'appui S est sensiblement parallèle à l'axe longitudinal L du boitier d'entrainement.

Selon l'exemple illustré la surface d'appui S est lisse et présente une forme concave qui lui permet de bien épouser la conformation des pommettes lorsque le massage est réalisé sous les yeux ou autour des yeux.

La tête de massage 1 comprend au-dessus de la surface d'appui S et à l'opposé du boîtier d'entraînement 2 par rapport à ladite surface d'appui S, les éléments de massage M qui comprennent au moins deux doigts de massage 21 qui comprennent chacun une tête de travail 22 destinée à venir au contact du visage.

Les deux doigts de massage 21 sont chacun mobiles entre, d'une part, une position de rétraction R correspondant à la position du doigt de massage 21 situé au premier plan à la figure 1 et, d'autre part, une position d'extension E correspondant à la position du doigt de massage 21 situé au second plan à la figure 1.

En position de rétraction R, la tête de travail 22 de chaque doigt de massage 21 se trouve en deçà de la surface d'appui S vers l'intérieur de la tête de massage 1. Tandis qu'en position d'extension E, la tête de travail 22 de chaque doigt de massage 21 se trouve au-delà de la surface d'appui S vers l'extérieur de la tête de massage 1. Chaque tête de travail 22 possède alors entre ses positions de rétraction R et d'extension E, une amplitude de déplacement a comprise entre 5 mm et 15 mm. Par ailleurs, en position d'extension E chaque tête de travail 22 est saillante par rapport à la surface d'appui S d'une distance d comprise 2 mm et 10 mm.

La tête de massage 1 comprend également des moyens de manoeuvre 25 adaptés pour déplacer alternativement chacun des doigts de massage 21 entre ses positions d'extension E et de rétraction R. Les moyens de manoeuvre 25 sont alors adaptés pour coopérer avec les moyens d'entraînement 7 et plus particulièrement avec l'arbre de sortie 8 de manière à transmettre et transformer le mouvement de rotation du moteur électrique 6 en un mouvement alternatif des doigts de massage 21.

Selon l'exemple illustré, chaque doigt de massage 21 présente une forme générale en S et est porté par un axe d'articulation 26 situé en partie basse du doigt de massage 21 correspondant tandis que la partie haute dudit doigt de massage 21 porte la tête de travail 22. Les moyens de manoeuvre comprennent alors une came 27 mobile en rotation sur elle-même selon une direction perpendiculaire à l'axe d'articulation 26 et coaxiale à l'arbre de sortie 8. La came 27 présente alors, au niveau d'une face inférieure, un logement de réception de l'arbre de sortie 8. La came 27 comprend, en outre, un chemin de came périphérique 28 dont le développé possède une forme sensiblement sinusoïdale. Chaque doigt de massage porte, à distance de l'axe d'articulation 26, un ergot 29 engagé dans le chemin de came 28 de sorte que la rotation de la came 27 sur elle-même induit un mouvement alternatif de chaque ergot 29. Compte tenu de la conformation des doigts de massage 21 le mouvement alternatif de chaque ergot 29 induit une oscillation alternative des doigts de massage en rotation autour de l'axe d'articulation 26.

De manière préférée, l'appareil de massage A et, plus particulièrement, les moyens de manoeuvre 25, les moyens d'entraînement 7, et l'unité de commande 10 sont adaptés pour assurer à chacun des doigts de massage une fréquence de déplacement supérieure ou égale à 2,5Hz.

Il doit être remarqué que, selon l'exemple illustré, les moyens de manoeuvre 25 sont adaptés pour coordonner le déplacement des doigts de massage 21 de manière que lorsque l'un des doigts de massage 21 est en position d'extension E, l'autre doigt de massage 21 est en position de rétraction R et réciproquement.

L'appareil de massage ainsi constitué est mis en oeuvre de la manière suivante. La surface d'appui S est placée contre le visage puis l'utilisateur met en marche l'appareil de massage A au moyen de l'interface 11, les doigts de massage 21 s'animent alors d'un mouvement d'oscillation qui reproduit le geste de massage qui serait effectué au moyen de deux doigts par exemple l'index et le majeur venant tapoter alternativement la peau du visage notamment celle se situant autour des yeux.

Selon l'exemple illustré et afin de simuler au mieux le geste des doigts, chaque tête de travail comprend une surface de travail T de forme convexe et dans le cas présent de forme sphérique et rigide.

Le massage réalisé au moyen de l'appareil selon l'invention procure un effet stimulant, un effet lissant, un effet relaxant et permet notamment de réduire les cernes ou les poches sous les yeux, et de combler les rides et ridules. Afin d'optimiser ce traitement, l'appareil de massage A tel qu'illustré aux figures 1 à 3 comprend des moyens 40 d'application ou de distribution de produit cosmétique. Selon l'exemple illustré, les moyens d'application de produits cosmétiques 40 comprennent un réservoir 41 situé dans le boitier d'entraînement 2 et raccordé, via un système de prélèvement 42, par exemple une pompe de prélèvement 42, à une buse de distribution 43 située dans l'élément d'appui 20. La pompe de prélèvement est pilotée par l'unité de commande 10 de manière à assurer la distribution de produit cosmétique lors du fonctionnement de l'appareil de massage A. Bien entendu, l'une des têtes de travail, voire les deux têtes de travail, pourrait comprendre une buse de distribution de produit cosmétique.

Par ailleurs, toujours selon l'exemple illustré aux figures 1 à 3, l'appareil de massage A comprend aussi des moyens d'application d'un courant électrique sur la peau 45 qui comprennent une unité 46 de génération d'un courant et/ou d'une tension électriques. L'unité de génération 46 est pilotée par l'unité de commande 10. L'unité de génération 46 est raccordée à une électrode 47 portée par une tête de travail 22.

Lors de l'utilisation de l'appareil de massage A, l'unité de commande 10 pilote le fonctionnement de l'unité de génération 46 de sorte que lorsque l'électrode 47 est en contact avec la peau un phénomène d'électrophorèse est induit qui favorise l'assimilation des principes actifs du produit cosmétique.

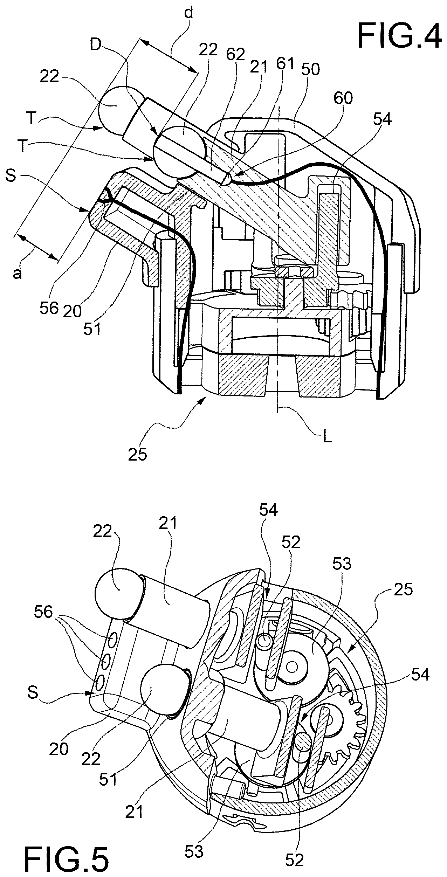

Selon l'invention, les doigts de massage 21 ne sont pas nécessairement animés d'un mouvement d'oscillation d'autour d'un axe horizontal. Ainsi les figures 4 et 5 montrent une tête de massage 1 pour un appareil selon l'invention dont les doigts de massage 21 possèdent une cinématique différente.

Selon cet exemple, chaque doigt de massage 21 est réalisé sous la forme d'une sorte de piston rectiligne qui s'étend en partie au moins à l'extérieur d'un corps creux 50 entourant les moyens de manoeuvre 25. Chaque doigt est alors guidé en translation par un alésage 51 aménagé dans le corps creux 50. L'extrémité de chaque doigt de massage 21 située à l'intérieur du corps creux 50 coopère avec un pion excentré 52 porté par un disque de manoeuvre 53 appartenant aux moyens de manoeuvre 25. Le pion excentré 52 est disposé dans une chambre 54 qui est liée rigidement au doigt de massage correspondant et dans laquelle le pion excentré 52 peut se déplacer en translation de sorte que sa rotation avec le disque de manoeuvre 53 est transformée en une translation du doigt de massage 21 correspondant.

De plus, selon cet exemple de réalisation, la surface d'appui S est plane.

Toujours selon cet exemple de réalisation, le débattement ou la course des doigts de massage n'est pas perpendiculaire à l'axe longitudinal L du boitier d'entrainement, mais forme un angle non nul avec ce dernier, selon l'exemple illustré un angle d'environ 30°. Ce mode de réalisation présente l'avantage de permettre à l'utilisateur d'atteindre plus facilement les zones étroites du visage comme le contour des yeux ou le contour de la bouche, sans être gêné par le corps de l'appareil.

Par ailleurs, selon cet exemple de réalisation, l'élément d'appui 20 porte des électrodes 56 de transmission d'un courant électrique à la peau.

Toujours selon cet exemple de réalisation, la tête de massage comprend des moyens 60 de diffusion de lumière en direction du visage. Dans le cas présent les moyens de diffusion 60 sont aménagés dans un doigt de massage 21 et comprennent une source de lumière 61 telle qu'une diode électroluminescente pilotée par l'unité de commande 10. La source de lumière 61 est alors associée à un système optique 62 comprenant une face de sortie D située au niveau de la surface de travail T et donc destinée à être orientée vers le visage de l'utilisateur de l'appareil de massage A selon l'invention.

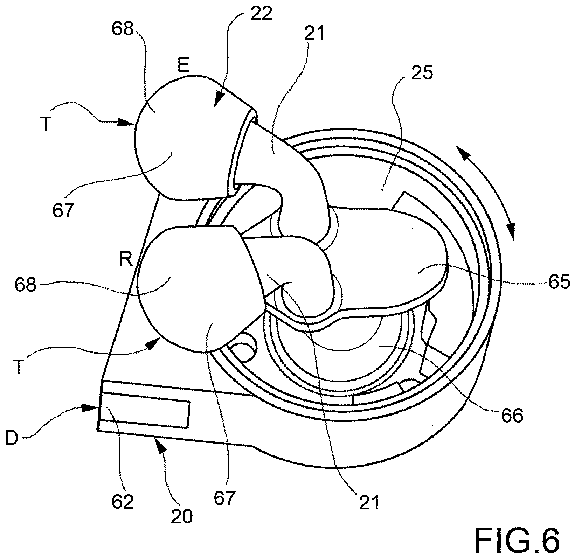

La figure 6 illustre encore une autre variante de réalisation d'une tête de massage 1 d'un appareil de massage A conforme à l'invention. Selon cette variante, les deux doigts de massage 21 sont portés par une même platine 65 qui oscille en rotation autour d'un axe vertical en étant entrainé, via un alésage présent sous la platine, par un disque à excentrique 66 appartenant aux moyens de manoeuvre 25 et dont le pion est disposé dans l'alésage de la platine. Selon cet exemple de réalisation, chaque doigt de massage 21 porte un capuchon amovible 67 qui forme la tête de travail 22 correspondante.

Selon un autre exemple de réalisation, ce capuchon peut être en matériau souple, type élastomère.

Selon un autre mode de réalisation, il peut comprendre un tampon 68 imbibé de produit cosmétique formant la surface de travail T. Les capuchons amovibles 67 forment ainsi des moyens d'application ou de distribution de produit cosmétique.

Selon cet exemple de réalisation, l'élément d'appui 20 comprend un système optique 62 de diffusion de la lumière produit par une source située soit dans la tête de massage 1 soit dans le boitier d'entrainement 2.

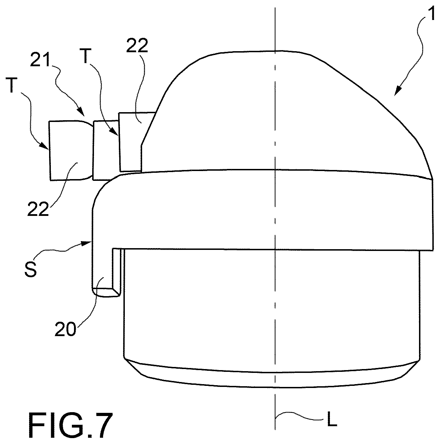

La figure 7 illustre une variante de réalisation de la tête de massage illustrée aux figures 4 et 5 dont elle partage la cinématique de manoeuvre des doigts de massage 21. Cette variante de réalisation diffère de la tête de massage illustrée figures 4 et 5 en ce que les doigts de massage se déplacent en translation alternative selon une direction perpendiculaire à l'axe longitudinal L du boitier d'entrainement. De plus, selon cette forme de réalisation, la surface de travail T de chaque doigt de massage est plane.

Bien entendu, diverses autres modifications ou variantes de l'appareil et de la tête de massage selon l'invention, peuvent être envisagées dans le cadre des revendications annexées.

## Revendications

1. Appareil de massage pour le visage comprenant :
- une tête de massage (1) qui comprend :
- un élément d'appui (20) destiné à venir contre le visage et qui définit une surface d'appui (S),
- au moins un doigt de massage (21), chacun comprenant une tête de travail (22) destinée à venir au contact du visage et chacun étant mobile entre :
- une position de rétraction (R) dans laquelle la tête de travail (22) est située en deçà de la surface d'appui (S) vers l'intérieur de la tête de massage (1),
- une position d'extension (E) dans laquelle la tête de travail (22) est située au delà de la surface d'appui (S) vers l'extérieur de la tête de massage (1),
- des moyens de manoeuvre (25) de chaque doigt de massage (21) adaptés pour déplacer chacun des doigts de massage (21) entre ses positions d'extension (E) et de rétraction (R) de manière alternative,
- et un boitier d'entraînement (2) qui porte la tête de massage (1) et qui comprend un moteur électrique (6) actionnant des moyens d'entrainement (7) adaptés pour transmettre le mouvement du moteur électrique (6) aux moyens de manoeuvre (25),
- **caractérisé en ce que** le boîtier d'entrainement (2) est situé à l'opposé des têtes de travail par rapport à l'élément d'appui (20).

2. Appareil de massage selon la revendication précédente, **caractérisé en ce que** la tête de massage (1) comprend au moins deux doigts de massage (21).

3. Appareil de massage selon la revendication précédente, **caractérisé en ce que** les moyens de manoeuvre (25) sont adaptés pour coordonner le déplacement des doigts de massage (21) de manière que lorsque l'un des doigts de massage (21) est en position d'extension (E) l'autre est en position de rétraction (R) et réciproquement.

4. Appareil de massage selon l'une des revendications précédentes, **caractérisé en ce que** l'élément d'appui (20) comprend une surface d'appui (S) concave ou plane.

5. Appareil de massage selon l'une des revendications précédentes, **caractérisé en ce que** l'élément d'appui (20) comprend une surface d'appui (S) lisse.

6. Appareil de massage selon l'une des revendications précédentes, **caractérisé en ce que** les doigts de massage (21) s'étendent en partie au moins à l'extérieur d'un corps creux (50) entourant en partie au moins les moyens de manoeuvre (25).

7. Appareil de massage selon l'une des revendications précédentes, **caractérisé en ce qu'**il est adapté pour assurer à chacun des doigts de massage (21) une fréquence de déplacement supérieure ou égale à 2,5 Hz.

8. Appareil de massage selon l'une des revendications précédentes, **caractérisé en ce que** chaque tête de travail (22) possède, entre sa position de rétraction (R) et sa position d'extension (E), une amplitude de déplacement (a) comprise entre 5 mm et 15 mm.

9. Appareil de massage selon l'une des revendications précédentes, **caractérisé en ce qu'**en position d'extension (E) chaque tête de travail (22) est saillante par rapport à la surface d'appui (S) d'une distance (d) comprise entre 2 mm et 10 mm.

10. Appareil de massage selon l'une des revendications précédentes, **caractérisé en ce que** chaque tête de travail (22) comprend une surface de travail (T) convexe ou plane.

11. Appareil de massage selon l'une des revendications précédentes, **caractérisé en ce que** chaque tête de travail (22) comprend une surface de travail (T) rigide et/ou en métal.

12. Appareil de massage selon l'une des revendications 1 à 9, **caractérisé en ce que** chaque tête de travail (22) comprend une surface de travail (T) élastiquement déformable.

13. Appareil de massage selon l'une des revendications précédentes, **caractérisé en ce qu'**il comprend des moyens (60) de diffusion de lumière en direction du visage.

14. Appareil de massage selon la revendication précédente, **caractérisé en ce que** les moyens de diffusion (60) de lumière comprennent au moins une source de lumière (61) et au moins un système optique (62) de diffusion comprenant une face (D) de sortie destinée à être orientée vers le visage.

15. Appareil de massage selon la revendication précédente, **caractérisé en ce que** l'élément d'appui (20) comprend une face (D) de sortie de la lumière.

16. Appareil de massage selon la revendication 14 ou 15, **caractérisé en ce que** l'une au moins des têtes de travail (22) comprend une face de sortie (D) de la lumière.

17. Appareil de massage selon l'une des revendications précédentes, **caractérisé en ce que** la tête de massage (1) est adaptée de manière amovible sur le boitier d'entrainement (2).

18. Appareil de massage selon la revendication précédente, **caractérisé en ce que** la tête de massage (1) comprend des moyens d'identification (13) et **en ce que** le boiter d'entrainement (2) comprend des moyens de reconnaissance (12) des moyens d'identification (13) raccordés à une unité de commande (10) adaptée pour contrôler le fonctionnement de l'appareil de massage en fonction de la tête de massage (1) reconnue.

## Patentansprüche

1. Massagegerät für das Gesicht, umfassend:
- einen Massagekopf (1), der umfasst:
- ein Stützelement (20), das dazu bestimmt ist, gegen das Gesicht zu drücken und das eine Stützfläche (S) definiert,
- mindestens einen Massagefinger (21), wobei jeder einen Arbeitskopf (22) umfasst, der dazu bestimmt ist, gegen das Gesicht zu drücken, und wobei jeder bewegbar ist zwischen:
- einer eingefahrenen Position (R), in der sich der Arbeitskopf (22) unterhalb der Stützfläche (S) in Richtung auf das Innere des Massagekopfes (1) befindet,
- einer ausgefahrenen Position (E), in der sich der Arbeitskopf (22) über der Stützfläche (S) nach außen von dem Massagekopf (1) befindet,
- Betätigungsmittel (25) von jedem Massagefinger (21), die eingerichtet sind, jeden der Massagefinger (21) zwischen seiner ausgefahrenen Position (E) und eingefahrenen Position (R) alternierend zu bewegen,
- und ein Antriebsgehäuse (2), das den Massagekopf (1) trägt und einen elektrischen Motor (6) umfasst, der Antriebsmittel (7) betätigt, die eingerichtet sind, die Bewegung des elektrischen Motors (6) auf die Betätigungsmittel (25) zu übertragen,
- **dadurch gekennzeichnet, dass** sich das Antriebsgehäuse (2) gegenüber Arbeitsköpfen mit Bezug auf das Stützelement (20) befindet.

2. Massagegerät nach dem vorhergehenden Anspruch, **dadurch gekennzeichnet, dass** der Massagekopf (1) wenigstens zwei Massagefinger (21) umfasst.

3. Massagegerät nach dem vorhergehenden Anspruch, **dadurch gekennzeichnet, dass** die Betätigungsmittel (25) eingerichtet sind, um die Verschiebung der Massagefinger zu (21) koordinieren, so dass, wenn einer der Massagefinger (21) in der ausgefahrenen Position (E) ist, der andere in der eingefahrenen Position (R) ist und umgekehrt.

4. Massagegerät nach einem der vorhergehenden Ansprüche, **dadurch gekennzeichnet, dass** das Stützelement (20) eine konkave oder planare Stütz fläche (S) umfasst.

5. Massagegerät nach einem der vorhergehenden Ansprüche, **dadurch gekennzeichnet, dass** das Stützelement (20) eine glatte Stütz fläche (S) umfasst.

6. Massagegerät nach einem der vorhergehenden Ansprüche, **dadurch gekennzeichnet, dass** sich die Massagefinger (21) zumindest teilweise außerhalb eines Hohlkörpers (50) erstrecken, der zumindest teilweise die Betätigungsmittel (25) umgibt.

7. Massagegerät nach einem der vorhergehenden Ansprüche, **dadurch gekennzeichnet, dass** es angepasst ist, für jeden der Massagefinger (21) eine Verschiebungsfrequenz bereitzustellen, die größer oder gleich 2,5 Hz ist.

8. Massagegerät nach einem der vorhergehenden Ansprüche, **dadurch gekennzeichnet, dass** jeder Arbeitskopf (22) zwischen seiner eingefahrenen Position (R) und seiner ausgefahrenen Position (E) eine Verschiebungsamplitude (a) aufweist, die zwischen 5 mm und 15 mm liegt.

9. Massagegerät nach einem der vorhergehenden Ansprüche, **dadurch gekennzeichnet, dass** in der ausgefahrenen Position (E) jeder Arbeitskopf (22) relativ zu der Stützfläche (S) um einen Abstand (d) hervorsteht, der zwischen 2 mm und 10 mm liegt.

10. Massagegerät nach einem der vorhergehenden Ansprüche, **dadurch gekennzeichnet, dass** jeder Arbeitskopf (22) eine konvexe oder planare Arbeitsfläche (T) umfasst.

11. Massagegerät nach einem der vorhergehenden Ansprüche, **dadurch gekennzeichnet, dass** jeder Arbeitskopf (22) eine steife und / oder metallische Arbeitsfläche (T) umfasst.

12. Massagegerät nach einem der Ansprüche 1 bis 9, **dadurch gekennzeichnet, dass** jeder Arbeitskopf (22) eine elastisch verformbare Arbeitsfläche (T) umfasst.

13. Massagegerät nach einem der vorhergehenden Ansprüche, **dadurch gekennzeichnet, dass** es Mittel (60) zur Streuung von Licht in der Richtung des Gesichts umfasst.

14. Massagegerät nach dem vorhergehenden Anspruch, **dadurch gekennzeichnet, dass** die Mittel (60) zur Streuung von Licht mindestens eine Lichtquelle (61) und mindestens ein optisches System (62) zur Streuung umfassen, das eine Fläche (D) zum Austritt des Lichts umfasst, die dazu bestimmt ist, auf das Gesicht gerichtet zu werden.

15. Massagegerät nach dem vorhergehenden Anspruch, **dadurch gekennzeichnet, dass** das Stützelement (20) eine Fläche (D) zum Austritt des Lichts umfasst.

16. Massagegerät nach Anspruch 14 oder 15, **dadurch gekennzeichnet, dass** mindestens einer der Arbeitsköpfe (22) eine Fläche (D) zum Austritt des Lichts umfasst.

17. Massagegerät nach einem der vorhergehenden Ansprüche, **dadurch gekennzeichnet, dass** der Massagekopf (1) abnehmbar an dem Antriebsgehäuse (2) angebracht ist.

18. Massagegerät nach dem vorhergehenden Anspruch, **dadurch gekennzeichnet, dass** der Massagekopf (1) Mittel zur Identifizierung (13) umfasst, und dass das Antriebsgehäuse (2) Mittel zum Erkennen (12) der Mittel zur Identifizierung (13) umfasst, die an einer Steuereinheit (10) angeschlossen sind, die eingerichtet ist, den Betrieb des Massagegerätes als Funktion des erfassten Massagekopfs (1) zu steuern.

## Claims

1. Facial massage apparatus comprising:
- a massage head (1) which comprises:
- a bearing element (20) intended to come against the face and which defines a bearing surface (S),
- at least one massage finger (21), each comprising a working head (22) intended to come into contact with the face and each being movable between:
-- a retraction position (R) wherein the working head (22) is situated short of the bearing surface (S) towards the inside of the massage head (1),
-- an extension position (E) wherein the working head (22) is situated beyond the bearing surface (S) towards the outside of the massage head (1),
- operating means (25) of each massage finger (21) suitable for moving each of the massage fingers (21) between the extension (E) and retraction (R) positions thereof in alternation,
- and a drive housing (2) which carries the massage head (1) and which comprises an electric motor (6) actuating drive means (7) suitable for transmitting the movement of the electric motor (6) to the operating means (25),
- **characterised in that** the drive housing (2) is situated opposite the working heads with respect to the bearing element (20).

2. Massage apparatus according to the preceding claim, **characterised in that** the massage head (1) comprises at least two massage fingers (21).

3. Massage apparatus according to the preceding claim, **characterised in that** the operating means (25) are suitable for coordinating the movement of the massage fingers (21) such that when one of the massage fingers (21) is in the extension position (E), the other is in the retraction position (R) and conversely.

4. Massage apparatus according to one of the preceding claims, **characterised in that** the bearing element (20) comprises a concave or planar bearing surface (S).

5. Massage apparatus according to one of the preceding claims, **characterised in that** the bearing element (20) comprises a smooth bearing surface (S).

6. Massage apparatus according to one of the preceding claims, **characterised in that** the massage fingers (21) extend in part at least to the outside of a hollow body (50) surrounding in part at least the operating means (25).

7. Massage apparatus according to one of the preceding claims, **characterised in that** it is suitable for providing each of the massage fingers (21) with a movement frequency greater than or equal to 2.5 Hz.

8. Massage apparatus according to one of the preceding claims, **characterised in that** each working head (22) has, between the retraction position (R) thereof and the extension position (E) thereof, a range of movement (a) between 5 mm and 15 mm.

9. Massage apparatus according to one of the preceding claims, **characterised in that**, in the extension position (E), each working head (22) projects with respect to the bearing surface (S) by a distance (d) between 2 mm and 10 mm.

10. Massage apparatus according to one of the preceding claims, **characterised in that** each working head (22) comprises a convex or planar working surface (T).

11. Massage apparatus according to one of the preceding claims, **characterised in that** each working head (22) comprises a rigid and/or metal working surface (T).

12. Massage apparatus according to one of claims 1 to 9, **characterised in that** each working head (22) comprises an elastically deformable working surface (T).

13. Massage apparatus according to one of the preceding claims, **characterised in that** it comprises light diffusion means (60) towards the face.

14. Massage apparatus according to the preceding claim, **characterised in that** the light diffusion means (60) comprise at least one light source (61) and at least one optical diffusion system (62) comprising an output surface (D) intended to be oriented towards the face.

15. Massage apparatus according to the preceding claim, **characterised in that** the bearing element (20) comprises a light output surface (D)

16. Massage apparatus according to claim 14 or 15, **characterised in that** at least one of the working heads (22) comprises a light output surface (D).

17. Massage apparatus according to one of the preceding claims, **characterised in that** the massage head (1) is removably fitted on the drive housing (2).

18. Massage apparatus according to the preceding claim, **characterised in that** the massage head (1) comprises identification means (13) and **in that** the drive housing (2) comprises means (12) for recognising the identification means (13) connected to a control unit (10) suitable for controlling the operation of the massage apparatus according to the massage head (1) recognised.
